# EUROPEAN PATENT SPECIFICATION

(11) **EP 3 485 799 B1**
(45) Date of publication and mention of the grant of the patent: **05.01.2022**
(21) Application number: 17202116.4
(22) Date of filing: 16.11.2017
(51) Int. Cl.: A61B 3/00, A61B 5/16, A61B 3/113, A61B 5/18

(54) **EYE TRACKING DEVICE**
AUGENVERFOLGUNGSVORRICHTUNG
DISPOSITIF DE SUIVI DES YEUX

(43) Date of publication of application: 22.05.2019
(73) Proprietor: Aptiv Technologies Limited, St. Michael (BB)
(72) Inventor: Polak, Andrzej, 50354 Huerth (DE)
(74) Representative: Manitz Finsterwald Patent- und Rechtsanwaltspartnerschaft mbB

(56) References cited:
- EP-A1- 1 908 396
- US-A1- 2015 237 246
- Sayani Ghosh ET AL: "Real Time Eye Detection and Tracking Method for Driver Assistance System" In: "Advancements of Medical Electronics", 1 January 2015 (2015-01-01), Springer India, New Delhi, XP055474368, ISSN: 2195-271X ISBN: 978-81-32-22256-9 pages 13-25, DOI: 10.1007/978-81-322-2256-9_2, * abstract; figures 1,2 * * sections "3. Proposed Scheme" and "4. Implementation" *

## Description

The present invention refers to an eye tracking device, in particular for the detection of a driver's visual behaviour.

Drowsiness and fatigue of an automobile driver may reduce the driver's abilities of vehicle control, natural reflex, recognition and perception. Such diminished vigilance level of a driver may for example be observed at night driving or caused by overdriving, finally leading to increased risks. It is known to employ driver assistance systems that can detect drowsiness and fatigue of the driver, in order to reduce the risks of accidents. Such driver assistance systems may use the eye tracking technology, since human eyes contain information about the person's condition such as gaze, attention level, and fatigue level.

The accuracy and robustness of eye tracking driver assistance systems depends on the consistency of image acquisition of the person's face in real time and under variable and complex background. For this purpose the person's face may be illuminated during image acquisition. However, illumination may cause light leaks between the respective illuminator and the imaging device, thereby limiting the performance of image acquisition.

Light leaks may in particular be caused by a cover for the illuminator and the imaging device, said cover being arranged primarily for improved appearance and protection of the illuminator and/or the imaging device. Light emitted by the illuminator may approach the cover at different angles and at least some rays may get trapped between bottom and upper surface of the cover due to internal reflections, finally leading to light leaks. This holds particularly true for arrangements, in which the illuminators are arranged close to the imaging device, for example, in order to achieve the so-called bright pupil effect. In order to prevent such light leaks, it is known to arrange shield elements made of light blocking material within the cover. This requires a two-component design of the cover, which is, however, difficult to manufacture and may cause an increased risk for visual defects within the cover, particularly due to weld lines, contaminations, sink marks and/or an unfavourable gating position. Such visual defects may again affect the performance and/or precision of image acquisition.

US 2015/237246 A1 discloses a state monitoring apparatus in a vehicle that monitors the state of a driver using a face image of the driver. This apparatus emits light from a light projecting part toward a predetermined area and receives light reflected by the face.

EP 1 908 396 A1 discloses an illumination and imaging system with glare reduction and a method therefor.

It is therefore an object of the present invention to provide an eye tracking device, which allows an improved image acquisition while at the same time allowing cost effective manufacturing, appropriate protection of the device's functional components as well as an appropriate appearance. It is also an object to provide a cover, preferably for such eye tracking device.

With regard to the eye tracking device, this objective has been solved by the subject matter of claim 1. A respective cover is subject of claim 15.

According to a first aspect of the present invention, an eye tracking device is particularly configured for the detection of a driver's visual behaviour. The eye tracking device is therefore preferably mountable within a vehicle, for example in the instrument panel, driving mirror or roof of a vehicle.

The eye tracking device according to the present teaching comprises an illuminator for illuminating a person's eye, an imaging device for detecting light reflected by the person's eye, and a cover for covering the illuminator and the imaging device. Furthermore, the cover comprises a light leak prevention structure for preventing light leaks between the illuminator and the imaging device through the cover, said light leak prevention structure being provided by and/or on at least a surface section of a cover portion being made of a material permeable to light emitted from the illuminator.

In the present teaching, the term light may generally visible light, thus light being visible to the human eye, and also light not visible to the human eye, for example electromagnetic radiation in the form of infrared light.

By providing an illuminator for the illumination of a person's eye, the impact of different ambient light conditions may be reduced, and hence the image quality generated by the imaging device may be ensured, also under varying real-world conditions including poor illumination, including different conditions at day and night.

Furthermore, by providing a light leak prevention structure by and/or on at least a surface section of a cover portion, the respective cover portion may be formed without interruptions by components or portions made of different material. That is, the light leak prevention is already ensured by and/or on the surface of the respective cover portion, such that the inner portions or the inner volume of the cover are not required to provide a shielding functionality. Accordingly, components or portions made of different material than the rest of cover may particularly be avoided within the inner volume of the cover. At least in portions being crucial to the optical characteristics of the cover, such as portions opposite to the imaging device, the inner volume of the cover may be free of components made of different material. Visual defects due to weld lines, contaminations, sink marks and/or unfavourable gating positions may thus be avoided.

The cover may have a one-component design or be continuously formed of a single material along portions being crucial to the optical characteristics of the cover. Components or layers made of different material, for example a light blocking material, may for example be arranged on the surface of the cover or used to provide a cover frame for holding and/or fixing the cover to a case or the like.

According to a preferred embodiment, the light leak prevention structure is provided by a shape, contour, orientation and/or texture of the respective surface section, namely the surface section of a cover portion being made of a material permeable to light emitted from the illuminator. Such surface may easily be provided during manufacturing of the cover, particularly by injection moulding. At the same time the light leak prevention structure being provided by a shape, contour, orientation and/or texture of the respective surface, a high level of functional safety may be ensured. Preferably, the shape, contour, orientation and/or texture of the respective surface section is different to the shape, contour, orientation and/or texture of an adjacent surface section. Thereby, a variation of the surface may be achieved, which allows a specific adaptation of the light leak prevention structure to the individual arrangement of components of the eye tracking device.

According to a further embodiment, the at least one surface section of the light leak prevention structure is a directing surface section, preferably configured to direct and/or refract light into directions away from the imaging device and/or into directions causing further reflections and/or refractions away from the imaging device. That is, the surface section may be configured and/or shaped to allow light to be directed and/or refracted into an appropriate direction, particularly in order to avoid light leaks from the illuminator through the cover into the imaging device. The respective rays are either directed and/or refracted away from the imaging device or the directing surface causes a redirecting and/or refraction into a direction which causes further reflections and/or refractions, which are advantageous for preventing light leaks. Such directing surface may achieve the respective light leak preventing functionality without the arrangement of additional light blocking elements or layers on the surface of the cover. Manufacturing may thereby be simplified.

According to yet another embodiment, the at least one surface section of the light leak prevention structure is provided on a protrusion and/or a recess of the respective cover section. A protrusion may have a curved, preferably convex, or angular, preferably prismatic and/or triangular, cross sectional shape. Accordingly, a recess may have a curved, preferably concave, or angular, preferably prismatic and/or triangular, cross sectional shape. Protrusions and recesses may be easily formed, for example, by injection moulding of the cover, while at the same time enabling a high degree of light leak prevention functionality.

According to a further embodiment, the light leak prevention structure comprises a light blocking layer arranged on and/or attached to the at least one surface section of the respective cover portion. The light blocking layer is preferably impermeable to light emitted from the illuminator. Thus, a light blocking layer may for example be attached to specifically defined portions of the cover, thereby preventing certain light rays to enter the interior of the cover. Internal reflections of these rays are thereby suppressed. Light blocking layers may form part of the light leak prevention structure. The arrangement of a light blocking layer does not exclude that the light leak prevention structure additionally comprises a surface section, which itself provides a light leak prevention functionality, such as a directing surface, for example. Thus, the light leak prevention structure may comprise a surface section, for example a directing surface, and in addition to this also a light blocking layer arranged and/or attached on a surface section, for example, a surface section adjacent to a directing surface.

According to yet a further embodiment, the at least one surface section of the light leak prevention structure extends along the respective cover portion in a linear and/or curved manner. In a plan view onto the respective surface section, said surface section may be curved or linear. Preferably, the protrusion and/or the recess may have a specific cross sectional shape. In a direction orthogonal to said cross sectional shape, the protrusion and/or the recess may have curved or linear shape. Dependant on the dimension and/or position of the illuminator and/or the imaging device, the light leak prevention may thereby be optimized.

In a further embodiment, the light leak prevention structure is provided by and/or on a plurality of surface sections, wherein said surface sections are preferably arranged symmetrically relative to the imaging device. Thus, different surface sections may be provided in view of different rays from the same and/or different light sources. Even in case of an intense illumination, for example by a large number of light sources, light leaks may be securely prevented.

According to yet a further embodiment, the at least one surface of the light leak prevention structure faces the illuminator and/or the imaging device and/or is formed on the side of the cover facing the illuminator and/or the imaging device. Thereby, the opposite surface of the cover, namely the surface facing away from the illuminator and/or the imaging device and/or facing the person's eye to be tracked, may be designed flat and/or smooth. The appearance of the cover may thereby be improved, as the person being subject to eye tracking by the eye tracking device will merely recognize a flat and/or smooth cover surface. However, it is also possible, that the light leak prevention structure comprises surface sections being arranged on the side of the cover facing away from the illuminator and/or the imaging device.

According to a further embodiment of the present invention, the cover has a varying thickness, preferably along the cover portion comprising the light leak prevention structure. Varying thicknesses may affect the path of rays inside the cover volume and therewith further improve the light leak prevention. Preferably, the cover may have a smaller thickness along a portion being opposite the imaging device than along adjacent portions. The distance between the surface of the cover portion opposite the imaging device and said imaging device may thereby be enhanced. Therefore, rays which exit the cover from a portion being opposite the imaging device are, dependent on the exit angle, less likely to enter the imaging device.

According to a further embodiment, the imaging device is configured to record the person's eye. Recording in the present context shall mean that data generated by the imaging device may be collected and/or saved. The data generated by the imaging device may be used for real time processing or also for processing with a certain time-delay. The imaging device may comprise a camera and/or a lens and/or an optical sensor. This allows a reliable detection of light reflected by the person's eye and/or reliable recording during operation.

The imaging device may be separated from the illuminator by a shield structure, which is impermeable to light emitted from the illuminator and/or sealed to the cover. More specifically, the light leak prevention structure may be configured to prevent light leaks through the cover around the shield structure. Accordingly, the shield structure may prevent rays from the illuminator to directly enter into the imaging device, thus, provides a shielding functionality. The light leak prevention structure may particularly be configured for preventing light leaks around said shield, namely through the cover into the imaging device.

The light leak preventing structure may be adapted to the dimensions and/or the position of the imaging device relative to the cover and/or adapted to the dimensions and/or position of the illuminator relative to the cover and/or to the dimensions and/or the position of the imaging device relative to the dimensions and/or the position of the illuminator. The specific design and/or arrangement of light leak preventing structure may therefore by chosen in view of the arrangement of the components of the eye tracking device. The light leak prevention may thereby be further optimized.

In a further embodiment, the illuminator is configured to emit infrared light, preferably near-infrared light. This allows producing the so-called bright pupil effect, which may further improve the detection and tracking of a person's eye, particular eye movements and/or blinks. That is, in case human eyes are illuminated with an infrared illuminator at certain wavelength, particularly a near-infrared illuminator, beaming light along the optical axis of the imaging device may cause a bright pupil. At the near-infrared wavelength, almost all infrared light is reflected from the pupils along the path back to the imaging device. Furthermore, near-infrared light is barely visible to the person subject to eye tracking and any interference with the person's activity, such as driving a vehicle, may be minimized.

According to a further embodiment, the illuminator comprises a plurality of light sources, preferably evenly and/or symmetrically arranged relative to the imaging device. The light sources may be designed as infrared LEDs. The light sources may be arranged close to the optical axis of the imaging device, particularly as close as possible in view of any geometrical restrictions.

According to another embodiment, the cover portion comprising the light leak prevention structure, in particular the entire cover, is permeable to infrared light, preferably near infrared light. Thereby, the cover may effectively protect the illuminator and/or the imaging device, without affecting illumination and/or detection by the imaging device. The cover may be impermeable to visible light. Thus, the cover may appear black or dark coloured to a person. Structures behind the cover will not be recognized by the person, whereby the overall appearance of the eye tracking device may be improved. The cover may be made of a plastic material, e.g. by injection moulding, which ensures low manufacturing costs and a reliable quality of the cover. The wall thickness of the cover may be chosen such that plastic flow during the injection process is enabled.

According to a second aspect of the present invention, a cover device is configured for an above described eye tracking device. The cover device comprises a cover for covering an illuminator and an imaging device arrangeable adjacent to the cover, and a light leak prevention structure for preventing light leaks between an illuminator and an imaging device through the cover, said light leak prevention structure being provided by and/or on at least a surface section of a cover portion being permeable to light emitted from the illuminator.

The further features mentioned above may likewise be applied to the cover device according to the second aspect of the present invention.

The present invention also refers to a driver assistant system including the above described eye tracking device and/or the above described cover device. An according driver assistant system may furthermore be equipped with a calculation unit such as a CPU and also a storage device, particularly for software. Such a system may enable eye tracking under variable and realistic lighting conditions and allows real-time acquisition of images of a driver's eye as well as processing and/or assessment of the acquired image data with improved performance.

The eye tracking device and/or the driver assistant system comprising such an eye tracking device may be configured as a stand-alone unit or as built-in unit.

The present invention is also directed to vehicles including an eye tracking device and/or a cover device and/or a driver assistance system mentioned above. The eye tracking device and/or the cover device may be installed in a vehicle dashboard, driving mirror and/or vehicle roof.

The features and advantages of the various embodiments of the present invention will, in the following, be described with reference to the figures.
- Fig. 1: shows a schematic cross sectional view of an eye tracking device according to an embodiment of the present invention;
- Fig. 2: shows a schematic cross sectional view of an eye tracking device according to the prior art without any light leak prevention portions;
- Fig. 3: shows a schematic cross sectional view of an eye tracking device according to the prior art with light leak prevention portions within the cover;
- Fig. 4a: shows a schematic perspective view of a cover of the eye tracking device in Fig. 3;
- Fig. 4b: shows the cover of Fig. 4a in a perspective cross sectional view;
- Fig. 5: shows a schematic cross sectional view of an eye tracking device according to a first embodiment of the present invention;
- Fig. 6: shows a schematic cross sectional view of an eye tracking device according to a second embodiment of the present invention;
- Fig. 7: shows a schematic cross sectional view of an eye tracking device according to a third embodiment of the present invention;
- Fig. 8: shows a schematic cross sectional view of an eye tracking device according to a fourth embodiment of the present invention;
- Fig. 9a: shows a schematic perspective view of a cover of an eye tracking device according to a fifth embodiment of the present invention;
- Fig. 9b: shows the cover of Fig. 9a in a perspective cross sectional view;
- Fig. 10: shows a schematic perspective view of a cover of an eye tracking device according to a sixth embodiment of the present invention;
- Fig. 11: shows a schematic plan view of a cover of an eye tracking device according to a seventh embodiment of the present invention.

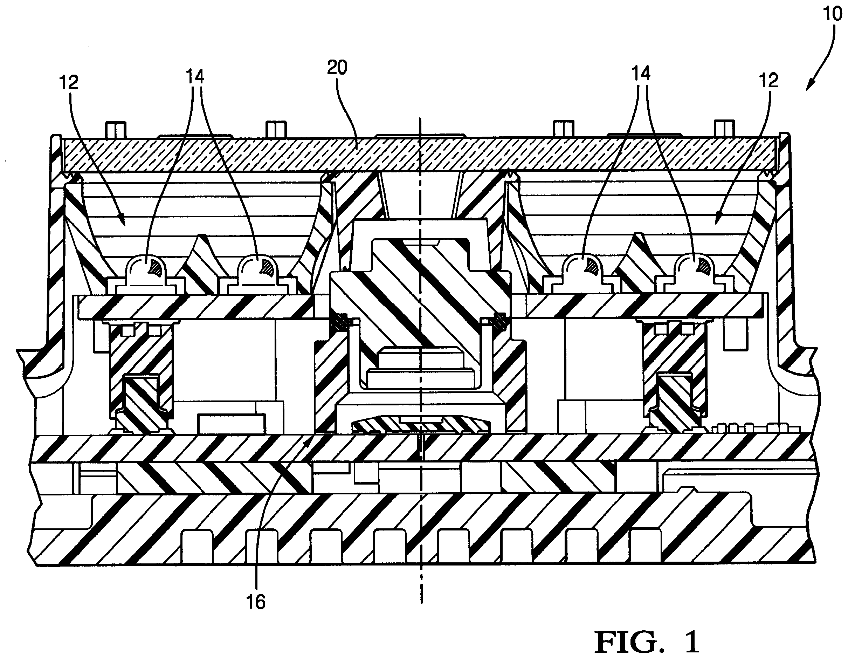

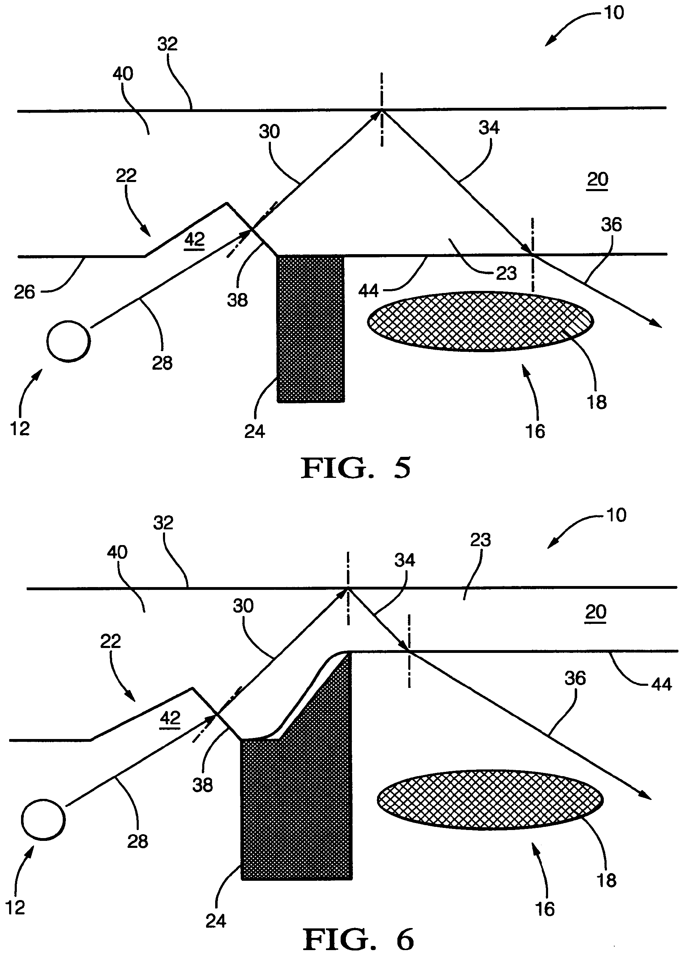

Fig. 1 shows a schematic cross sectional view of an eye tracking device 10 according to an embodiment of the present invention. The eye tracking device comprises an illuminator 12 for illuminating a person's eye, the person's eye not being shown here. The illuminator 12 may consist of a plurality of light sources 14. The light sources 14 may be configured for emitting infrared light, more preferably near infrared light. The eye tracking device 10 also comprises an imaging device 16 for detecting light reflected by a person's eye. The imaging device 16 may comprise a lens 18, which is best shown schematically in Fig. 5 to 8.

Furthermore, the eye tracking device 10 in Fig. 1 comprises a cover 20 for covering the illuminator 12 and the imaging device 16. According to the present invention, the cover 20 comprises a light leak prevention structure 22 for preventing light leaks between the illuminator 12 and the imaging device 16 through the cover 20. The light leak prevention structure 22 will be described in more detail with reference to Fig. 5 to 11 below.

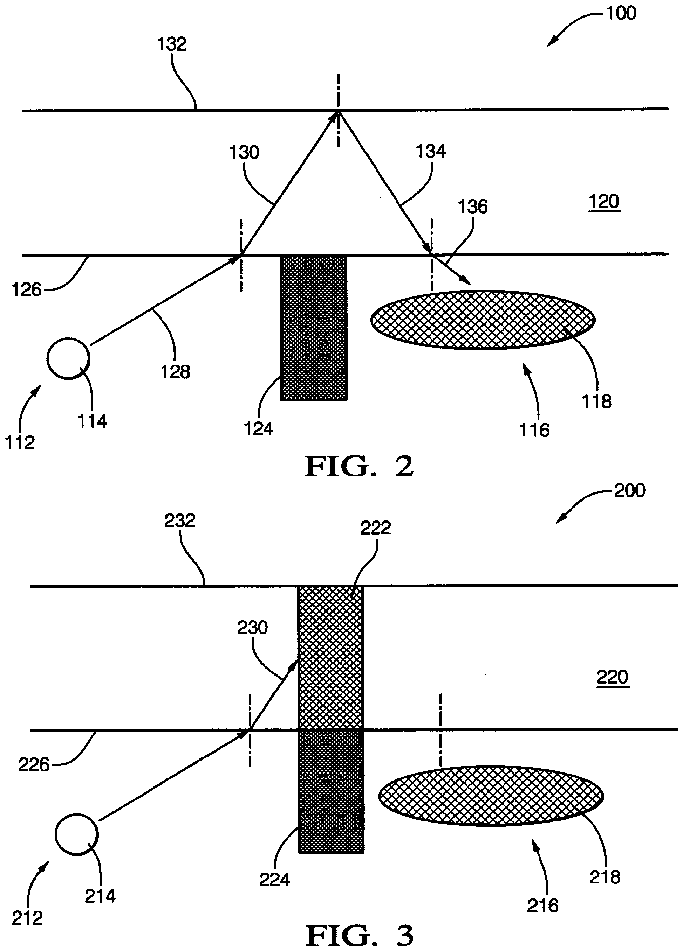

Fig. 2 shows a schematic cross sectional view of an eye tracking device 100 according to the prior art without any light leak prevention portions. Fig. 2 illustrates the problem of so-called light leaks. As may be comprehended from Fig. 2, the eye tracking device 100 comprises an illuminator 112, an imaging device 116 including a lens 118 as well as a cover 120. A shield element 124 is provided between the illuminator 112 and the imaging device 116 in order to prevent rays from the illuminator 112 to directly enter into the imaging device 116. As may be comprehended from Fig. 2, light rays may enter the cover 120 from a bottom surface 126 in a direction indicated with numeral 128 at a certain angle and thereby be refracted into a direction indicated with numeral 130 inside the cover 120. In the following, the rays, at least to a certain extent, may be reflected back from the upper surface 132 of the cover 120 into a direction indicated with numeral 134. The rays, when exiting the cover 120 on the bottom surface 126 may be again refracted into a direction indicated with numeral 136. In direction 136 the rays may enter the lens 118 of the imaging device 116. Light leaks caused by such refractions and reflections of the rays, as described above, may affect the performance of the imaging device 116.

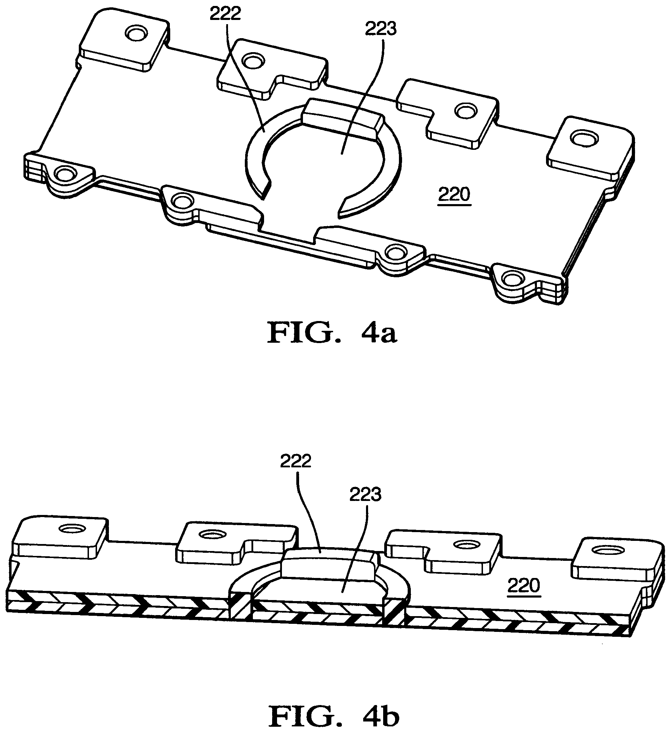

It is known to prevent such light leaks with an eye tracking device 200 shown in Fig. 3. The difference of the eye tracking device 200 in comparison to the eye tracking device 100 of Fig. 2 is that the cover 220 is equipped with a light leak prevention structure 222. The light leak prevention structure 222 is made of a light blocking material and in-moulded into the cover 220. That is, the cover 220 is made of two different materials, and therefore has a so called two-component design. Rays from illuminator 212 entering the bottom surface 226 in a direction indicated with numeral 228 are refracted into the direction 230 within the cover 220. These rays are then blocked by the light leak prevention structure 222, either before being reflected from the upper surface 232 or before. Light leaks between the illuminator 212 and the imaging device 216 may thus be prevented with structure 222. Figs. 4a and 4b show perspective views of the cover 220 of Fig. 3.

However, the two-component design of the cover 220 of Fig. 3, 4a and 4b is difficult to manufacture, and may also cause increased risk for visual defects within the cover 220, particularly due to weld lines, contaminations, sink marks and/or an unfavourable gating position. Such visual defects may particularly occur along cover portions 223 of the cover 220 being arranged opposite the imaging device 216. This is due to the fact that cover portion 223 is bordered by the light leak prevention structure 222 made of a different material. During manufacturing weld lines may for example not be avoided in portion 223. Visual defects caused thereby may affect the performance and/or precision of image acquisition by the imaging device 216.

In the following reference is made to Fig. 5 to 11, showing different embodiments of the present invention. Fig. 5 shows an eye tracking device 10 according to a first embodiment of present invention. As mentioned above, the eye tracking device 10 comprises an illuminator 12, an imaging device 16 with a lens 18, and a cover 20 for covering the illuminator 12 and the imaging device 16. The cover 20 is provided with a light leak prevention structure 22 being provided by and/or on at least a surface section of a cover portion being made of a material permeable to light emitted from the illuminator 12. The illuminator 12 and the imaging device 16 are separated by a shield structure 24.

Due to the light leak prevention structure 22, light rays emitted by the illuminator 12 in the direction indicated by numeral 28 enter the cover 20 from the bottom side 26 at a different angle, which is different to the angle shown in Fig. 2 and 3, and therefore are also the refraction of the rays is differently. Namely, by entering the rays are refracted into a direction indicated by numeral 30 and then, at least partly, reflected back from the upper surface 32 into a direction indicated with numeral 34. When exiting the interior of the cover 20 on the bottom side 26, the rays are again refracted, namely into a direction indicated by numeral 36. It may be comprehended that the rays in direction 36 do not enter the lens 18 of the imaging device 16, and therefore may not affect imaging performance.

In the embodiment of Fig. 5, the light leak prevention structure 22 is provided by at least a surface section 38 of a cover portion 40 with a recess 42. The surface section 38 of the recess 42 forms a directing surface, which causes a specific refraction of rays, finally leading said rays away from the lens 18. Particularly, the surface section 38 may have a different orientation than adjacent surface sections and/or angled relative to adjacent surface sections. Furthermore, the light leak prevention structure 22 may also comprise a surface section 44 on cover portion 23 opposite the lens 18. That is, the surface section 44 from which the respective rays exit the interior of the cover 20 may also be part of the light leak prevention structure 22.

The embodiment of Fig. 6 differs from the embodiment in Fig. 5 merely in the thickness of the cover 20 along the portion 23 opposing the lens 18 of the imaging device 16. That is, the portion 23 of the cover opposing the lens 18 of the imaging device 16 has a smaller thickness than adjacent portions of the cover 20. Therefore, the distance of the surface 44 of the portion 23 to the lens 18 is increased as compared to the embodiment in Fig. 5. Accordingly, the refraction of rays exiting the cover 20 on the surface section 44 of cover portion 23 into the direction indicated with numeral 36 occurs with greater distance to the lens 18, and the risk of rays reaching the lens 18 is reduced.

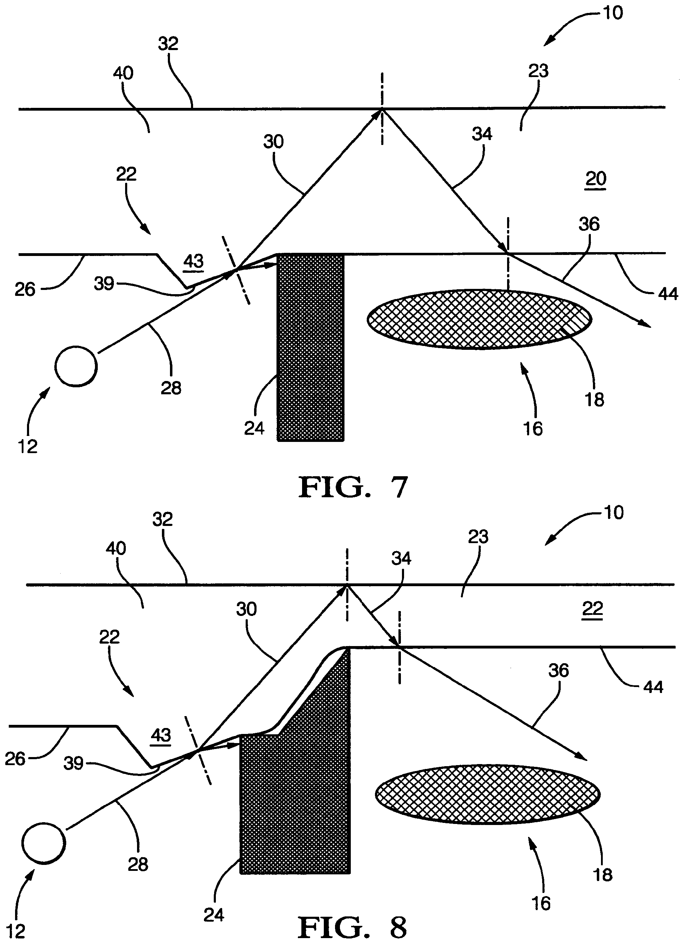

In the embodiment of Fig. 7, the light leak prevention structure 22 is provided by at least a surface section 39 of a cover portion 40 with a protrusion 43. The surface section 39 of the protrusion 43 forms a directing surface, which causes a specific refraction of rays, finally leading said rays away from the lens 18. Also in this embodiment, the light leak prevention structure 22 may comprise a surface section 44 on cover portion 23 opposite the lens 18. The surface section 44 from which the respective rays exit the interior of the cover 20 may also be part of the light leak prevention structure 22.

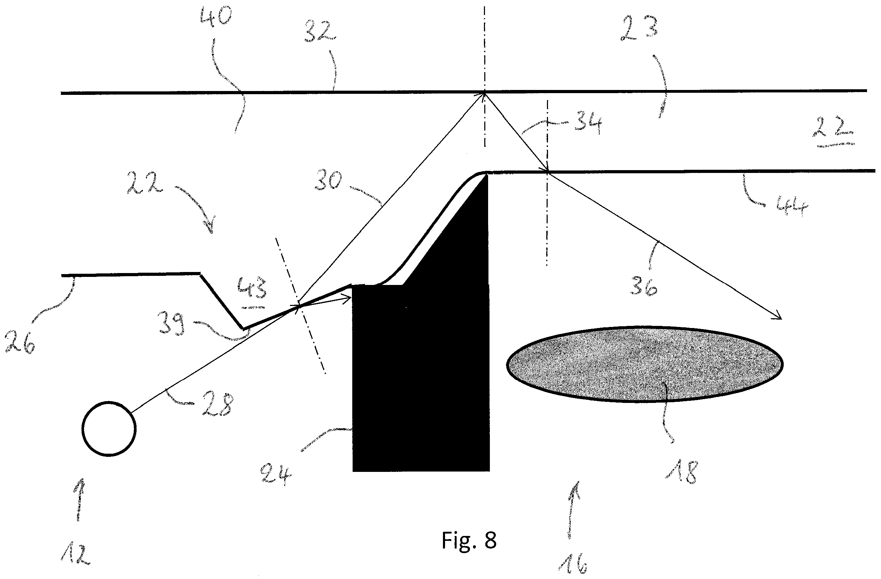

The embodiment of Fig. 8 differs from the embodiment in Fig. 7 merely in the thickness of the cover 20 along the portion 23 opposing the lens 18 of the imaging device 16. The portion 23 of the cover 20 opposing the lens 18 of the imaging device 16 has a smaller thickness than adjacent portions of the cover 20. Therefore, the distance of the surface 44 of the portion 23 to the lens 18 is increased as compared to the embodiment in Fig. 7. Accordingly, the refraction of rays exiting the cover 20 on the surface section 44 of cover portion 23 into the direction indicated with numeral 36 occurs with greater distance to the lens 18, and the risk of rays reaching the lens 18 is reduced.

The cross-sectional shape of the recess 42 and/or of the protrusion 43 may also be different to the illustrations in Figs. 5 to 8. That is, the recess 42 and/or the protrusion 43 may also have rounded surfaces 38 or 39, respectively. In other words, the recess 42 may, for example, be concave in cross-section. The protrusion 43 may also be convex in cross-section.

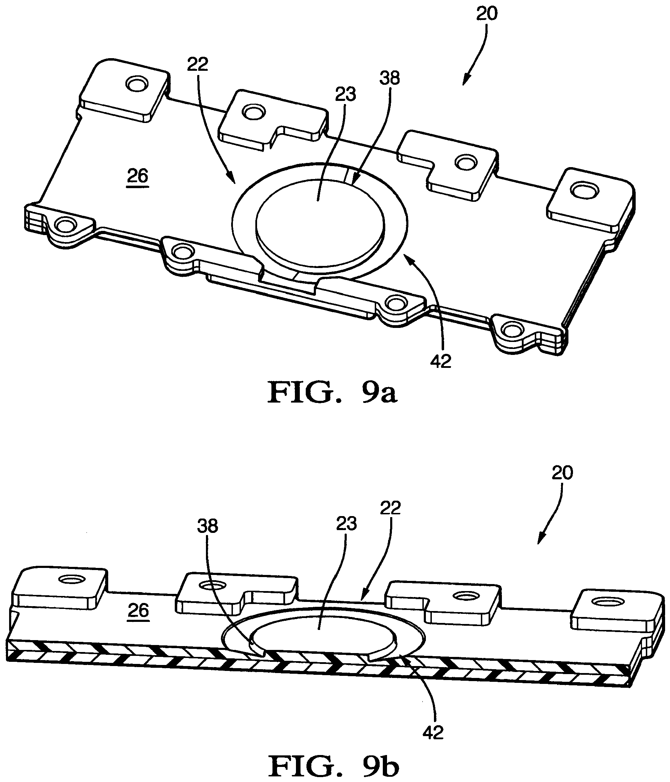

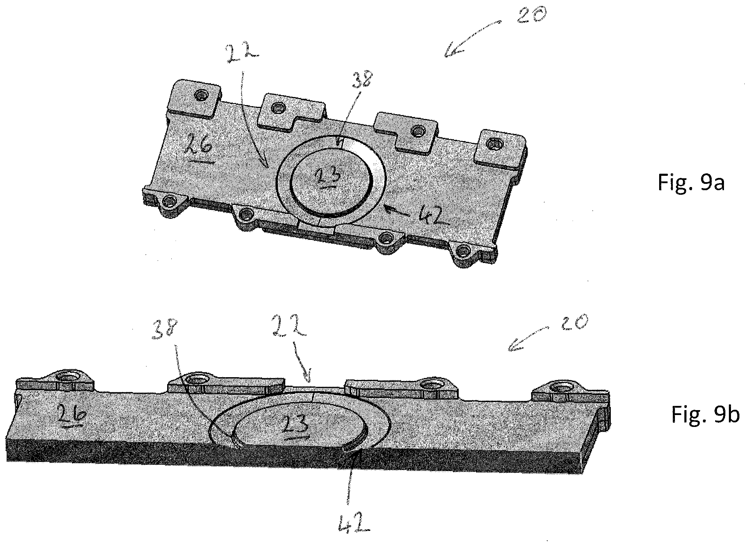

Figs. 9a and 9b show schematic perspective views of a cover 20 of an eye tracking device according to a further embodiment. The cover 20 in Figs. 9a and 9b comprises a light leak prevention structure 22, which is provided by at least a surface section 38 of a cover portion 40 with a recess 42, which is also in accordance with the embodiment in Fig. 5. As may be comprehended Figs. 9a and 9b, the recess 42 has a circular shape in a plan view on bottom side 26 the cover 20. That is, the cover recess 42 encircles the portion 23, which may be arranged opposite to a lens 18 of an imaging device 16.

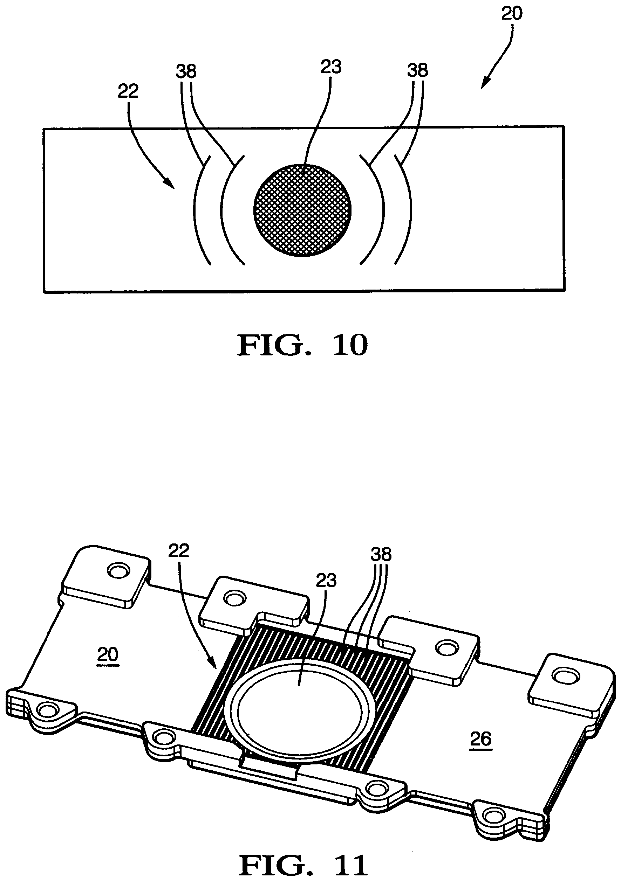

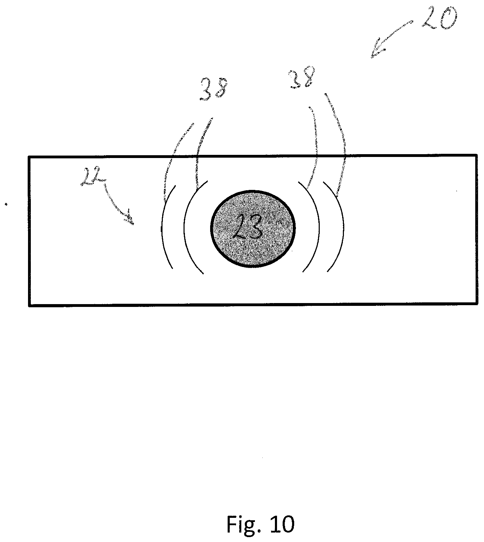

Fig. 10 shows a schematic plan view of a cover 20 according to a further embodiment. The cover 20 in Fig. 10 differs from the embodiment in Figs. 9a and 9b merely in that the light leak prevention structure 22 is provided by a plurality of surface sections 38. These surface sections 38 may be arranged symmetrically around a portion 23 of the cover 20, which may be arranged opposite to a lens 18 of an imaging device 16.

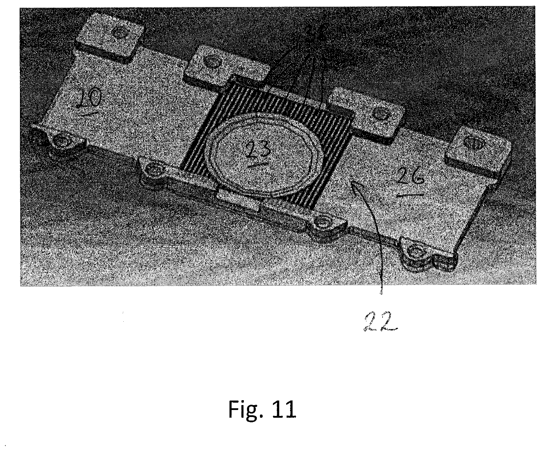

Fig. 11 shows a schematic perspective view of a cover 20 according to a further embodiment. The cover 20 in Fig. 11 differs from the embodiment in Fig. 10 in that the light leak prevention structure 22 is provided by a plurality of surface sections 38, which extend linearly along the bottom surface 26 of the cover. The surface sections 38 may be arranged parallel to each other and/or extend orthogonal to the length axis of the cover. The accumulated number of surface sections 38 may enclose a portion 23 of the cover 20, which may be arranged opposite to a lens 18 of an imaging device 16.

In the embodiments of Fig. 5 to 11, the light leak prevention structure 22 may also be provided on at least a surface section of a cover portion 40 being made of a material permeable to light emitted from an illuminator 12. For example, a light blocking material layer may be attached to surface sections of the cover 20. Such light blocking material layer may be provided instead or in addition to the surface section 38 or 39, as described above. A light blocking material layer may be provided on surface sections 38 or 39 described above. In other words, in addition to or instead of directing surfaces, such as surface section 38 or 39 as described above, the light leak preventing structure 22 may comprise masked surface sections, which allows further improved light leak prevention.

### List of reference signs

- 10: eye tracking device
- 12: illuminator
- 14: light source
- 16: imaging device
- 18: lens
- 20: cover
- 22: light leak prevention structure
- 23: cover portion
- 24: shield structure
- 26: bottom surface
- 28-36: ray direction
- 38, 39: surface section
- 40: cover portion
- 42: recess
- 43: protrusion
- 44: surface section
- 100, 200: eye tracking device
- 112,212: illuminator
- 114, 214: light source
- 116,216: imaging device
- 118,218: lens
- 120,220: cover
- 222: light leak prevention structure
- 124, 224: shield structure
- 126,226: bottom surface
- 128-136: ray direction
- 228-236: ray direction

## Claims

1. Eye tracking device (10), in particular for the detection of a driver's visual behaviour, comprising an illuminator (12) for illuminating a person's eye, an imaging device (16) for detecting light reflected by the person's eye, and a cover (20) for covering the illuminator (12) and the imaging device (16), the cover comprising a light leak prevention structure (22) for preventing light leaks between the illuminator (12) and the imaging device (16) through the cover (20), said light leak prevention structure (22) being provided by at least a surface section (38, 39) of a cover portion (40) being made of a material permeable to light emitted from the illuminator (12).

2. Eye tracking device (10) according to claim 1, wherein the light leak prevention structure (22) is provided by a shape, contour, orientation and/or texture of the respective surface section (38, 39), which is preferably different to the a shape, contour, orientation and/or texture of an adjacent surface section.

3. Eye tracking device (10) according to claim 1 or 2, wherein the at least one surface section (38, 39) of the light leak prevention structure (22) is a directing surface section, preferably configured to direct and/or refract light into directions away from the imaging device (16) and/or into directions causing further reflections and/or refractions away from the imaging device (16).

4. Eye tracking device (10) according to at least one of the preceding claims, wherein the at least one surface section (38, 39) of the light leak prevention structure is provided on a protrusion (43) and/or a recess (42) of the respective cover section (40) and/or wherein the protrusion (43) has a curved, preferably convex, or angular, preferably prismatic and/or triangular, cross sectional shape and/or wherein the recess (42) has a curved, preferably concave, or angular, preferably prismatic and/or triangular, cross sectional shape.

5. Eye tracking device (10) according to at least one of the preceding claims, wherein the light leak prevention structure (22) comprises a light blocking layer arranged on and/or attached to the at least one surface section of the respective cover portion, wherein the light blocking layer is impermeable to light emitted from the illuminator.

6. Eye tracking device (10) according to at least one of the preceding claims, wherein the at least one surface section (38, 39) of the light leak prevention structure (22), preferably the protrusion (43) and/or the recess (42), extends along the respective cover portion (40) in a linear and/or curved manner.

7. Eye tracking device (10) according to at least one of the preceding claims, wherein the light leak prevention structure (22) is provided by and/or on a plurality of surface sections (38, 39), wherein said surface sections (38, 39) are preferably arranged symmetrically relative to the imaging device (16).

8. Eye tracking device (10) according to at least one of the preceding claims, wherein the at least one surface section (38, 39) of the light leak prevention structure (22) faces the illuminator (12) and/or the imaging device (16) and/or is formed on the side (26) of the cover (20) facing the illuminator (12) and/or the imaging device (16).

9. Eye tracking device (10) according to at least one of the preceding claims, wherein the cover (20) has a varying thickness, preferably along the cover portion (40) comprising the light leak prevention structure, and/or wherein the cover has a smaller thickness along a portion (23) being opposite the imaging device (16) than along adjacent portions.

10. Eye tracking device (10) according to at least one of the preceding claims, wherein the imaging device (16) is configured to record the person's eye and/or comprises a camera and/or a lens (18) and/or an optical sensor.

11. Eye tracking device (10) according to at least one of the preceding claims, wherein the imaging device (16) is separated from the illuminator (12) by a shield structure (24), which is impermeable to light emitted from the illuminator (12) and/or sealed to the cover (20), wherein preferably the light leak prevention structure (22) is configured to prevent light leaks through the cover (20) around the shield structure (24).

12. Eye tracking device (10) according to at least one of the preceding claims, wherein the light leak preventing structure (22) is adapted to the dimensions and/or the position of the imaging device (16) relative to the cover (20) and/or adapted to the dimensions and/or position of the illuminator (12) relative to the cover (20) and/or to the dimensions and/or the position of the imaging device (16) relative to the dimensions and/or the position of the illuminator (12).

13. Eye tracking device (10) according to at least one of the preceding claims, wherein the illuminator (12) is configured to emit infrared light, preferably near-infrared light, and/or wherein the illuminator (12) comprises a plurality of light sources (14), preferably symmetrically arranged relative to the imaging device (16).

14. Eye tracking device (10) according to at least one of the preceding claims, wherein the cover portion (40) comprising the light leak prevention structure (22), preferably the entire cover (20), is permeable to infrared light, preferably near infrared light, and/or impermeable to visible light and/or wherein the cover (20) is made of a plastic material, preferably injection moulded.

15. Cover device, preferably for an eye tracking device (10) according to at least one of the preceding claims, comprising a cover (20) for covering an illuminator (12) and an imaging device (16) arrangeable adjacent to the cover (20), and a light leak prevention structure (22) for preventing light leaks between an illuminator (12) and an imaging device (16) through the cover (20), said light leak prevention structure (22) being provided by at least a surface section (38, 39) of a cover portion being permeable to light emitted from the illuminator (12).

## Patentansprüche

1. Augenverfolgungsvorrichtung (10), insbesondere für die Erfassung des Sehverhaltens eines Fahrers, mit einer Beleuchtungseinrichtung (12) zum Beleuchten des Auges einer Person, einer Bildgebungsvorrichtung (16) zum Erfassen des von dem Auge der Person reflektierten Lichts und einer Abdeckung (20) zum Abdecken der Beleuchtungseinrichtung (12) und der Bildgebungsvorrichtung (16), wobei die Abdeckung eine Lichtleckverhinderungsstruktur (22) zum Verhindern von Lichtlecks zwischen der Beleuchtungseinrichtung (12) und der Bildgebungsvorrichtung (16) durch die Abdeckung (20) aufweist, wobei die Lichtleckverhinderungsstruktur (22) durch mindestens einen Oberflächenabschnitt (38, 39) eines Abdeckabschnitts (40) bereitgestellt ist, der aus einem Material hergestellt ist, das für von der Beleuchtungseinrichtung (12) emittiertes Licht durchlässig ist.

2. Augenverfolgungsvorrichtung (10) nach Anspruch 1, wobei die Lichtleckverhinderungsstruktur (22) durch eine Form, Kontur, Ausrichtung und/oder Textur des jeweiligen Oberflächenabschnitts (38, 39) bereitgestellt ist, die sich vorzugsweise von der Form, Kontur, Ausrichtung und/oder Textur eines benachbarten Oberflächenabschnitts unterscheidet.

3. Augenverfolgungsvorrichtung (10) nach einem der Ansprüche 1 oder 2, wobei der mindestens eine Oberflächenabschnitt (38, 39) der Lichtleckverhinderungsstruktur (22) ein lenkender Oberflächenabschnitt ist, der vorzugsweise so konfiguriert ist, dass er Licht in Richtungen weg von der Bildgebungsvorrichtung (16) und/oder in Richtungen lenkt und/oder bricht, die weitere Reflexionen und/oder Brechungen weg von der Bildgebungsvorrichtung (16) verursachen.

4. Augenverfolgungsvorrichtung (10) nach einem der vorhergehenden Ansprüche, wobei der mindestens eine Oberflächenabschnitt (38, 39) der Lichtleckverhinderungsstruktur an einem Vorsprung (43) und/oder einer Ausnehmung (42) des jeweiligen Abdeckabschnitts (40) vorgesehen ist und/oder wobei der Vorsprung (43) eine gekrümmte, vorzugsweise konvexe, oder eckige, vorzugsweise prismatische und/oder dreieckige, Querschnittsform aufweist und/oder wobei die Ausnehmung (42) eine gekrümmte, vorzugsweise konkave oder eckige, vorzugsweise prismatische und/oder dreieckige, Querschnittsform aufweist.

5. Augenverfolgungsvorrichtung (10) nach einem der vorhergehenden Ansprüche, wobei die Lichtleckverhinderungsstruktur (22) eine lichtblockierende Schicht umfasst, die auf dem mindestens einen Oberflächenabschnitt des jeweiligen Abdeckabschnitts angeordnet und/oder daran befestigt ist, wobei die lichtblockierende Schicht für von der Beleuchtungseinrichtung emittiertes Licht undurchlässig ist.

6. Augenverfolgungsvorrichtung (10) nach einem der vorhergehenden Ansprüche, wobei sich der mindestens eine Flächenabschnitt (38, 39) der Lichtleckverhinderungsstruktur (22), vorzugsweise der Vorsprung (43) und/oder die Ausnehmung (42) linear und/oder gekrümmt entlang des jeweiligen Abdeckabschnitts (40) erstreckt.

7. Augenverfolgungsvorrichtung (10) nach einem der vorhergehenden Ansprüche, wobei die Lichtleckverhinderungsstruktur (22) durch und/oder auf einer Vielzahl von Oberflächenabschnitten (38, 39) vorgesehen ist, wobei die Oberflächenabschnitte (38, 39) vorzugsweise symmetrisch relativ zu der Bildgebungsvorrichtung (16) angeordnet sind.

8. Augenverfolgungsvorrichtung (10) nach einem der vorhergehenden Ansprüche, wobei der mindestens eine Oberflächenabschnitt (38, 39) der Lichtleckverhinderungsstruktur (22) der Beleuchtungseinrichtung (12) und/oder der Bildgebungsvorrichtung (16) zugewandt ist und/oder an der der Beleuchtungseinrichtung (12) und/oder der Bildgebungsvorrichtung (16) zugewandten Seite (26) der Abdeckung (20) ausgebildet ist.

9. Augenverfolgungsvorrichtung (10) nach einem der vorhergehenden Ansprüche, wobei die Abdeckung (20) eine variierende Dicke, vorzugsweise entlang des Abdeckabschnitts (40) aufweist, der die Lichtleckverhinderungsstruktur umfasst, und/oder wobei die Abdeckung entlang eines Abschnitts (23), der der Bildgebungsvorrichtung (16) gegenüberliegt, eine geringere Dicke als entlang benachbarter Abschnitte aufweist.

10. Augenverfolgungsvorrichtung (10) nach einem der vorhergehenden Ansprüche, wobei die Bildgebungsvorrichtung (16) zur Aufnahme des Auges der Person ausgebildet ist und/oder eine Kamera und/oder eine Linse (18) und/oder einen optischen Sensor umfasst.

11. Augenverfolgungsvorrichtung (10) nach einem der vorhergehenden Ansprüche, wobei die Bildgebungsvorrichtung (16) von der Beleuchtungseinrichtung (12) durch eine Abschirmstruktur (24) getrennt ist, die für von der Beleuchtungseinrichtung (12) ausgestrahltes Licht undurchlässig und/oder gegenüber der Abdeckung (20) abgedichtet ist, wobei vorzugsweise die Lichtleckverhinderungsstruktur (22) so konfiguriert ist, dass sie Lichtlecks durch die Abdeckung (20) um die Abschirmstruktur (24) herum verhindert.

12. Augenverfolgungsvorrichtung (10) nach einem der vorhergehenden Ansprüche, wobei die Lichtleckverhinderungsstruktur (22) an die Abmessungen und/oder die Position der Bildgebungsvorrichtung (16) relativ zur Abdeckung (20) angepasst ist und/oder an die Abmessungen und/oder die Position der Beleuchtungseinrichtung (12) relativ zur Abdeckung (20) und/oder an die Abmessungen und/oder die Position der Bildgebungsvorrichtung (16) relativ zu den Abmessungen und/oder der Position der Beleuchtungseinrichtung (12) angepasst ist.

13. Augenverfolgungsvorrichtung (10) nach einem der vorhergehenden Ansprüche, wobei die Beleuchtungseinrichtung (12) so konfiguriert ist, dass sie Infrarotlicht, vorzugsweise Nahinfrarotlicht, emittiert, und/oder wobei die Beleuchtungseinrichtung (12) eine Vielzahl von Lichtquellen (14) umfasst, die vorzugsweise symmetrisch relativ zu der Bildgebungsvorrichtung (16) angeordnet sind.

14. Augenverfolgungsvorrichtung (10) nach einem der vorhergehenden Ansprüche, wobei der die Lichtleckverhinderungsstruktur (22) aufweisende Abdeckabschnitt (40), vorzugsweise die gesamte Abdeckung (20), für infrarotes Licht, vorzugsweise Licht aus dem nahen Infrarot, durchlässig und/oder für sichtbares Licht undurchlässig ist und/oder wobei die Abdeckung (20) aus einem Kunststoffmaterial, vorzugsweise aus Spritzguss, hergestellt ist.

15. Abdeckvorrichtung, vorzugsweise für eine Augenverfolgungsvorrichtung (10) nach einem der vorhergehenden Ansprüche, mit einer Abdeckung (20) zum Abdecken einer Beleuchtungseinrichtung (12) und einer Bildgebungsvorrichtung (16), die benachbart zu der Abdeckung (20) anordenbar ist, und einer Lichtleckverhinderungsstruktur (22) zum Verhindern von Lichtlecks zwischen einer Beleuchtungseinrichtung (12) und einer Bildgebungsvorrichtung (16) durch die Abdeckung (20), wobei die Lichtleckverhinderungsstruktur (22) durch mindestens einen Oberflächenabschnitt (38, 39) eines Abdeckabschnitts vorgesehen ist, der für von der Beleuchtungseinrichtung (12) emittiertes Licht durchlässig ist.

## Revendications

1. Dispositif de suivi oculaire (10), en particulier pour la détection d'un comportement visuel d'un conducteur, comprenant un moyen d'illumination (12) destiné à illuminer un œil d'une personne, un dispositif d'imagerie (16) destiné à détecter une lumière réfléchie par l'œil de la personne, et une couverture (20) destinée à couvrir le moyen d'illumination (12) et le dispositif d'imagerie (16), la couverture comprenant une structure d'empêchement de fuite de lumière (22) pour empêcher les fuites de lumière entre le moyen d'illumination (12) et le dispositif d'imagerie (16) à travers la couverture (20), ladite structure d'empêchement de fuite de lumière (22) étant assurée par moins une section de surface (38, 39) d'une portion de couverture (40) faite d'un matériau perméable à la lumière émise depuis le moyen d'illumination (12).

2. Dispositif de suivi oculaire (10) selon la revendication 1, dans lequel la structure d'empêchement de fuite de lumière (22) est assurée par une forme, un contour, une orientation et/ou une texture de la section de surface (38, 39) respective, qui est de préférence différent(e) d'une forme, d'un contour, d'une orientation et/ou d'une texture d'une section de surface adjacente.

3. Dispositif de suivi oculaire (10) selon la revendication 1 ou 2, dans lequel ladite au moins une section de surface (38, 39) de la structure d'empêchement de fuite de lumière (22) est une section de surface de direction, de préférence configurée pour diriger et/ou réfracter la lumière dans des directions en éloignement du dispositif d'imagerie (16) et/ou dans des directions amenant des réflexions et/ou réfractions supplémentaires en éloignement du dispositif d'imagerie (16).

4. Dispositif de suivi oculaire (10) selon l'une au moins des revendications précédentes,
dans lequel ladite au moins une section de surface (38, 39) de la structure d'empêchement de fuite de lumière est prévue sur une projection (43) et/ou un évidement (42) de la section de couverture (40) respective et/ou dans lequel la projection (43) a une forme de section transversale incurvée, de préférence convexe, ou angulaire, de préférence prismatique et/ou triangulaire, et/ou dans lequel l'évidement (42) a une forme de section transversale incurvée, de préférence concave, ou angulaire, de préférence prismatique et/ou triangulaire.

5. Dispositif de suivi oculaire (10) selon l'une au moins des revendications précédentes, dans lequel la structure d'empêchement de fuite de lumière (22) comprend une couche de blocage de lumière agencée et/ou fixée sur ladite au moins une section de surface de la portion de couverture respective, dans lequel la couche de blocage de lumière est imperméable à la lumière émise depuis le moyen d'illumination.

6. Dispositif de suivi oculaire (10) selon l'une au moins des revendications précédentes, dans lequel ladite au moins une section de surface (38, 39) de la structure d'empêchement de fuite de lumière (22), de préférence la projection (43) et/ou l'évidement (42), s'étend le long de la portion de couverture (40) respective d'une manière linéaire et/ou incurvée.

7. Dispositif de suivi oculaire (10) selon l'une au moins des revendications précédentes, dans lequel la structure d'empêchement de fuite de lumière (22) est assurée par et/ou prévue sur une pluralité de sections de surface (38, 39), dans lequel lesdites sections de surface (38, 39) sont de préférence agencées de manière symétrique relativement au dispositif d'imagerie (16).

8. Dispositif de suivi oculaire (10) selon l'une au moins des revendications précédentes, dans lequel ladite au moins une section de surface (38, 39) de la structure d'empêchement de fuite lumière (22) fait face au moyen d'illumination (12) et/ou au dispositif d'imagerie (16) et/ou est formée sur le côté (26) de la couverture (20) faisant face au moyen d'illumination (12) et/ou au dispositif d'imagerie (16).

9. Dispositif de suivi oculaire (10) selon l'une au moins des revendications précédentes, dans lequel la couverture (20) a une épaisseur variable, de préférence le long de la portion de couverture (40) comprenant la structure d'empêchement de fuite de lumière, et/ou dans lequel la couverture a une épaisseur plus petite le long d'une portion (23) qui est opposée au dispositif d'imagerie (16) que le long de portions adjacentes.

10. Dispositif de suivi oculaire (10) selon l'une au moins des revendications précédentes, dans lequel le dispositif d'imagerie (16) est configuré pour enregistrer le regard de la personne et/ou comprend une caméra et/ou une lentille (18) et/ou un capteur optique.

11. Dispositif de suivi oculaire (10) selon l'une au moins des revendications précédentes, dans lequel le dispositif d'imagerie (16) est séparé du moyen d'illumination (12) par une structure formant écran (24), qui est imperméable à la lumière émise depuis le moyen d'illumination (12) et/ou est scellée à la couverture (20), dans lequel de préférence la structure d'empêchement de fuite de lumière (22) est configurée pour empêcher les fuites de lumière à travers la couverture (20) autour de la structure formant écran (24).

12. Dispositif de suivi oculaire (10) selon l'une au moins des revendications précédentes, dans lequel la structure d'empêchement de fuite de lumière (22) est adaptée aux dimensions et/ou à la position du dispositif d'imagerie (16) relativement à la couverture (20) et/ou adaptée aux dimensions et/ou à la position du moyen d'illumination (12) relativement à la couverture (20) et/ou aux dimensions et/ou à la position du dispositif d'imagerie (16) relativement aux dimensions et/ou à la position du moyen d'illumination (12).

13. Dispositif de suivi oculaire (10) selon l'une au moins des revendications précédentes, dans lequel le moyen d'illumination (12) est configuré pour émettre une lumière infrarouge, de préférence une lumière proche de l'infrarouge, et/ou dans lequel le moyen d'illumination (12) comprend une pluralité de sources de lumière (14), de préférence agencées symétriquement relativement au dispositif d'imagerie (16).

14. Dispositif de suivi oculaire (10) selon l'une au moins des revendications précédentes, dans lequel la portion de couverture (40) comprenant la structure d'empêchement de fuite de lumière (22), de préférence la couverture (20) entière, est perméable à la lumière infrarouge, de préférence à la lumière proche de l'infrarouge, et/ou est imperméable à la lumière visible et/ou dans lequel la couverture (20) est faite d'un matériau en plastique, de préférence moulé par injection.

15. Dispositif de couverture, de préférence pour un dispositif de suivi oculaire (10) selon l'une au moins des revendications précédentes, comprenant une couverture (20) destinée à couvrir un moyen d'illumination (12) et un moyen d'imagerie (16) pouvant être agencée de manière adjacente à la couverture (20), et une structure d'empêchement de fuite de lumière (22) destinée à empêcher les fuites de lumière entre un moyen d'illumination (12) et un dispositif d'imagerie (16) à travers la couverture (20), ladite structure d'empêchement de fuite de lumière (22) étant assurée par au moins une section de surface (38, 39) d'une portion de couverture qui est perméable à la lumière émise depuis le moyen d'illumination (12).
